(19) Europäisches Patentamt
European Patent Office
Office européen des brevets

(11) **EP 4 316 510 A1**

(12) **EUROPEAN PATENT APPLICATION**
published in accordance with Art. 153(4) EPC

(43) Date of publication:
**07.02.2024 Bulletin 2024/06**

(21) Application number: **22718850.5**

(22) Date of filing: **16.03.2022**

(51) International Patent Classification (IPC):
**A61K 39/00** (2006.01)    **A61P 37/00** (2006.01)
**C07K 14/535** (2006.01)

(52) Cooperative Patent Classification (CPC):
**A61K 39/0008; A61K 39/001139; C07K 14/535;**
A61K 2039/575; A61K 2039/6031; A61K 2039/6068

(86) International application number:
**PCT/CU2022/050002**

(87) International publication number:
**WO 2022/207016 (06.10.2022 Gazette 2022/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.03.2021  CU 20210021**

(71) Applicant: **Centro de Inmunologia Molecular
Playa, La Habana 11300 (CU)**

(72) Inventors:
• **LAGE DÁVILA, Agustín Bienvenido**
  **La Habana 10400 (CU)**
• **LEDÓN NARANJO, Nuris**
  **La  Habana 11500 (CU)**
• **PEREIRA YÁÑEZ, Karla**
  **La Habana 11000 (CU)**

• **SILVA SOSA, Alexa**
  **La Habana 10700 (CU)**
• **FUENTES MORALES, Dasha**
  **La Habana 10800 (CU)**
• **SAAVEDRA HERNANDEZ, Danay**
  **La Habana 10800 (CU)**
• **OTERO ALFARO, Oscar**
  **La Habana 17100 (CU)**
• **SUÁREZ FORMIGO, Gisela María**
  **La Habana 10400 (CU)**
• **ROJAS DORANTES, Gertrudis**
  **La Habana 10700 (CU)**
• **PÉREZ MARTÍNEZ, Dayana**
  **La Habana 10800 (CU)**
• **GALVEZ VALCARCEL, Jesus Ramón**
  **La Habana 10600 (CU)**
• **LÓPEZ MEDIANILLA, Armando**
  **La Habana 17100 (CU)**

(74) Representative: **Capitán García, Maria Nuria
Felipe IV no. 10, bajo iz.
28014 Madrid (ES)**

(54) **VACCINE COMPOSITIONS DEPLETING HEMATOPOIETIC GROWTH FACTORS FOR THE TREATMENT OF INFLAMMATORY DISEASES**

(57)    The present invention is related to the fields of Biotechnology and Medicine. Particularly, it describes therapeutic vaccine compositions able to produce an autoimmune reaction against haemopoietic growth factors such as G-SCF and/or GM-CSF bounded to other molecules or a fragment thereof by chemical conjugation or fusion. Such vaccines compositions are useful for the treatment of inflammatory diseases, especially wherein a pathological increasing of the circulating neutrophils occurs.

EP 4 316 510 A1

**Description**

TECHNIQUE FIELD

The present invention is related to the branches of Biotechnology and Medicine. It particularly describes vaccine compositions whose antigen is a hematopoietic growth factor that can be granulocyte colony-stimulating factor and/or granulocyte and monocyte colony-stimulating factor.

PREVIOUS ART

Inflammation is a complex process, whereby leukocytes and plasma-derived effector proteins are recruited to specific tissue sites to drive a local immune response (Newton K, Dixit VM. (2012) Cold Spring Harb Perspect Biol. 4 (3): a006049). Although it is an effective way to limit infections and initiate tissue remodeling, it must be controlled to avoid collateral tissue damage. Short periods of acute inflammation allow infection or injury to be contained and wound healing, but prolonged periods of chronic inflammation (due to activation of cytokine-producing cells and granulocytes to increase their production, creating a positive feedback loop) can cause increased tissue damage locally and systemically regulated immunity, particularly T-cell responses.

Chronic inflammation for various reasons lacks a complete resolution phase, it never ends. There are different reasons for this, such as prolonged contact with infections or irritants and the presence of cells that continually secrete inflammatory mediators. When the inflammatory stimulus becomes permanent, immunosuppression and immunological tolerance begin. In this case, subclinical but elevated levels of cytokines such as granulocyte colony-stimulating factor (G-CSF) and granulocyte-monocyte colony-stimulating factor (GM-CSF) (Granulocyte-macrophage colony-stimulating factor) are related to immune tolerance, while significantly elevated levels are related to inflammatory exacerbations (Rogovskii V. (2020) Front Immunol. 11:2061). In fact, inflammatory cytokines, such as G-CSF and GM-CSF, may mediate increased immune tolerance. The price of increased immune tolerance is increased susceptibility to tumors (Stape T (2018) Asia Pac J Oncol Nurs 5:40-2).

G-CSF and GM-CSF are the major cytokines in granulopoiesis and differentiation of normal granulocytic precursors in the bone marrow. Its physiological effects are mediated by binding to specific cell surface receptors. Plasma concentrations of G-CSF and GM-CSF and their receptors are known to be altered in inflammatory diseases showing neutrophilia, fever, inflammation, tissue destruction, and in some cases shock and death (Watari K et al. (1989 ) Blood.73(1): 117-22; Hamilton JA (2020) J Exp Med.217(1):e20190945).

When an infection occurs, the release of G-CSF and GM-CSF increases naturally because some components of the infectious agent stimulate their production. The neutrophils originated from the chain of reactions produced, in turn, attack the infectious agents, favoring their destruction. (Eyles JL et al. (2006) Nat. Clin. Pract. Rheumatol. 2(9): 500-510; Ahandideh B et al. (2020) Hum Immunol. 81(5):206-217). G-CSF and GM-CSF also have a key role in cancer initiation, progression, and metastasis (Do H et al. (2020) Cancers. 12(2):287). The combined action of cytokines, produced by neoplastic cells, modulates the cellular response of the host's immune system. High levels of inflammatory cytokines have been correlated with advanced stage and poor prognosis for several types of cancer (Silva EM et al. (2017) PLoS ONE 12(7): e0181125. ; Lippitz, BE (2013).; The Lancet Oncology, 14(6), e218-e228).

Neutrophils are known to play a significant role in different pathologies, which are characterized by chronic inflammation, such as the development of tumors, chronic obstructive pulmonary disease, uveitis, arthritis, ankylosing spondylitis, lupus erythematosus, asthma, syndrome cytokine release, among others. The neutrophil/lymphocyte ratio is a prognostic indicator in several of them (Lee HN et al. (2019) Rheumatol Int. 39(5):859-868).

In the clinic, G-CSF and GM-CSF have been used extensively to treat chemotherapy-associated neutropenia and to mobilize hematopoietic stem cells for transplantation (Roberts, AW (2005) Growth Factors 23(1): 33- 4; Mehta HM et al. (2015) J. Immunol. 195(4):1341-1349), however, we have not found precedents to the contrary.

It has been described monoclonal antibody (Abs) that can play a diagnostic role or as passive therapy to combat tumors with high expression of the G-CSF receptor (G-CSFR). A humanized neutralizing monoclonal Ab directed against G-CSFR has been reported that be well tolerated in primates and did not result in neutropenia. In addition, a murine anti-G-CSFR monoclonal Ab suppressed arthritis and significantly inhibited neutrophil accumulation in joints, which occurred without neutropenia, suggesting that G-CSFR blockade affects neutrophil localization to inflammatory sites. (Campbell IK (2016). J. Immunol. 197: 4392-4402). However, no evidence has been found for antiproliferative and antitumor treatment. Since G-CSF is a key ligand in the control and function of neutrophils, the use of formulations that inhibit the activation of this cytokine as therapeutic indications in patients with this type of disease is useful and important. There is a phase I study in healthy volunteers with a monoclonal anti-G-CSFR Ab for the treatment of hidradenitis suppurative and palmoplantar pustulosis (WO 2019/178645 and WO 2020/113270), where the number of circulating neutrophils does not drop to lower levels. critics.

The blockade of the GM-CSF pathway by monoclonal Abs directed against the cytokine itself or its receptor is described in WO 2010/124163, and there are ongoing clinical trials in patients with refractory rheumatoid arthritis https://clinicaltrials.gov/ct2/show/NCT04333147, Accessed: November 23, 2020; ClinicalTrials.gov. https://clinicaltrials.gov/ct2/show/NCT04134728, Accessed: Nov 23, 2020) and in COVID patients https://clinicaltrials.gov/ct2/show/NCT04376684, Accessed: November 23, 2020. It has also been described the use of Abs against GM-CSF in the treatment of toxicity induced by ACT-type immunotherapies (WO 2019/070680).

The concentrations of G-CSF and GM-CSF cytokines that are detected in patients are high, so even high doses of antibodies are unable to completely neutralize their activity. Breaking tolerance and generating immunogenicity are not obvious processes for these molecules because they are found in the body under normal conditions. Until now, no active immunotherapy against G-CSF or GM-CSF-dependent tumors, capable of inhibiting their growth, has been proposed.

For the first time, in the present invention, the depleting immune response of these hematopoietic growth factors is presented, and evidence of the therapeutic effect of this response in experimental models of inflammation and cancer is shown. Said compositions produce an increase in anti-GCSF and anti-GM-CSF Ab titers, a decrease in the number of circulating neutrophils, an antiproliferative, anti-inflammatory effect, and a high antitumor effect. In addition, they can be used in chronic scenarios because by requiring low doses of the active ingredients, their toxicity is reduced.

The effects shown by the vaccine compositions described herein are surprising and unexpected. The complex nature of the cytokine networks and their pleiotropic effects and the redundancy of the mechanisms that generate inflammation do not make it obvious that the Abs generated by these vaccine compositions can selectively depress the population of neutrophils, much less that this depletion translates into in an anti-inflammatory and anti-tumor effect.

BRIEF DESCRIPTION OF THE INVENTION

In one procedure, action the object of the present invention is a therapeutic vaccine composition to induce an immune response against hematopoietic growth factors that comprises a carrier protein, an adjuvant, and at least one antigen that can be rG-CSF or rGM-CSF.

Among the carrier proteins used by these vaccine compositions are cholera toxin B, tetanus toxoid, KLH, Neisseria meningitides P64k, diphtheria toxoid, peptides that are capable of presenting T epitopes against G-CSF and GM-CSF, immunoglobulin G, immunoglobulin M, Fc region of antibodies, variable fragments of antibodies, and proteins from bacteria, yeast, or mammals. These carrier proteins can be attached to the antigen by chemical conjugation or fusion methods.

The adjuvants that can be used in the vaccine compositions of the present invention are incomplete Freund's adjuvant, complete Freund's adjuvant, adjuvants based on squalene, adjuvants of synthetic origin, adjuvants of mineral origin, adjuvants of vegetable origin, adjuvants of animal protein, particulate protein adjuvants, proteoliposome-type adjuvants, liposomes, and mixtures of any of the foregoing.

In a particular procedure, action, the present invention is related to the use of the vaccine compositions described herein in the treatment of inflammatory diseases that are selected from a group that includes: cancer, chronic obstructive pulmonary disease, uveitis, rheumatoid arthritis, ankylosing spondylitis, lupus erythematosus, Crohn's disease, asthma, dermatitis, cytokine release syndrome, and diseases where cell degranulation plays an important role.

In another procedure, action, a method of treating a subject in need is described, comprising the administration of a therapeutically effective amount in a range between 0.01 and 10 mg/kg of weight of the vaccine compositions of the present invention. Particularly, an immune response induction stage produced between 1 and 6 doses administered at least weekly is carried out, and another for maintaining said immune response between 1 dose and until use-limiting toxicity occurs, administered at least weekly. This method comprises the administration of the vaccine compositions either by the intramuscular, subcutaneous, or intratumoral route.

DETAILED DESCRIPTION OF THE INVENTION

**Vaccine compositions**

The present invention comprises obtaining vaccine preparations to induce an immune response against the hematopoietic growth factors G-CSF and GM-CSF, which also produce a decrease in the number of circulating neutrophils, an antiproliferative effect, and a high antitumor effect in vivo.

The active principle of the vaccine compositions of the present invention is a protein conjugate between the antigen (G-CSF and/or GM-CSF) bound to a carrier protein. Said carrier protein is selected from but not limited to cholera toxin B, tetanus toxoid, KLH, P64k from Neisseria meningitidis; diphtheria toxoid and peptides that are capable of presenting T epitopes against G-CSF and GM-CSF. In the above conjugates, the protein can be fused to immunoglobulins G, immunoglobulins M, the Fc region of Abs, whether human or from another animal species; said Fc region can be a variant of limited binding to Fc receptors consisting of an IgG1 mutated in the region Cy2 with mutations L234A and L235A. In addition, the conjugates can be fused to variable fragments of Abs, bacterial, yeast, or mammalian proteins.

Additionally, the protein conjugates described above comprise an adjuvant, which enhances or complements their anti-G-CSF and/or anti-GM-CSF Abs response. These adjuvants can be incomplete Freund's adjuvant, complete Freund's adjuvant, squalene-based adjuvants, adjuvants of synthetic origin, adjuvants of mineral origin, adjuvants of plant origin, adjuvants of animal origin, particulate protein adjuvants, proteoliposome-type adjuvants, liposomes, or a mixture of any of the above.

The anti-G-CSF and/or anti-GM-CSF systems comprised in the present invention use suitable pharmaceutical excipients. These include, but are not limited to: water for injection, sodium chloride, phosphorous and potassium salts, calcium chloride, sodium hydroxide and citrate, and EDTA. Patients can be inoculated in parenteral formulations with protein concentrations from 0.01 to 10 mg/mL and doses between 10-100 $\mu$L/kg or 10-100 $\mu$g of total protein per kilogram or up to 5 mg of total protein, being more recommended 10-60 $\mu$g/kg.

The therapeutic composition can be stored using its liquid form at temperatures between -80°C and 8°C or after a lyophilization process at temperatures between 2-8°C.

**Obtaining the chemical conjugate between the carrier protein and recombinant G-CSF (rG-CSF) and/or recombinant G-CSF (rGM-CSF)**

From the evaluation and optimization of the conditions for the chemical conjugation reaction between the rGCSF and/or rGM-CSF proteins and some of the carrier proteins mentioned above, a chemical conjugation method is developed that requires a high molar ratio of the autologous protein, to guarantee a high conjugation efficiency between both during conjugation. The necessary excess of autologous protein during conjugation is then eliminated through the ultrafiltration membrane purification method, which allows obtaining the vaccine preparation with high homogeneity.

The procedure described in the present invention guarantees an adequate chemical conjugation between both proteins and consists of a single step. It begins by mixing the previously concentrated rGCSF and/or rGM-CSF proteins in a range between 0.1 and 1 mg/mL and the molecule to which it will be conjugated in a range between 0.6 and 20 mg/mL in the conjugation reactor. The PBS/MgCl2 solution (pH 6.0-7.2) and the glutaraldehyde conjugation solution in a range between 0.1-0.8% are subsequently added to this protein mixture. The mixture is kept under constant stirring between 15 minutes and 4 hours at a temperature between 20 and 24°C $\pm$ 2°C. The total protein concentration during the conjugation reaction is 1-20 mg/mL

Subsequently, purification is carried out using ultrafiltration membranes in a range between 50-100kDa and consisting of two stages. In the initial stage, successive changes of buffer solution (diafiltration) are performed to remove glutaraldehyde and eliminate excess autologous protein, either free or forming conjugates of only the rGCSF protein or only the rGM-CSF protein of different sizes. During this stage, between 3 and 15 changes of buffer solution are performed. The second stage is the concentration of the purified chemical conjugate.

The therapeutic preparation obtained by membrane purification is characterized by a chemical conjugation ratio between the rGCSF proteins and the molecule to which it is conjugated in a range between 5:1 to 20:1, and free of glutaraldehyde.

**Obtaining the therapeutic composition by the fusion protein expression method**

The vaccine compositions of the present invention can also be obtained by designing genetic constructions based on the rG-CSF and rGM-CSF genes cloned in an expression vector, preferably PCMX, without being limited to it, and fused to the genes of any of the aforementioned carrier proteins. The cells used in the transfection can be HEK-293T, HEK-293-GE, Expi 293, HEK-293, or CHOk1. The supernatants of the transfected cells are collected after 6-10 days of culture. Said recombinant proteins are then purified by protein A affinity chromatography or metal ion affinity chromatography.

**Treatment methods**

The systems described above are effective in maintaining the anti-G-CSF and/or anti-GM-CSF immune response, with prior induction of Abs generated by the systems described in this invention. They are useful in the treatment of inflammatory diseases where G-CSF and GM-CSF play a relevant role. These diseases include (but are not limited to) cancer (especially those tumors dependent on G-CSF or GM-CSF), chronic obstructive pulmonary disease, uveitis, arthritis, ankylosing spondylitis, lupus erythematosus, Crohn's, asthma, dermatitis, cytokine release syndrome, and in diseases where cell degranulation plays an important role.

The doses approved for use in humans or animals will be administered between 1 and 6 induction doses, administered at least weekly, and then in a stage of maintenance of the immunological response, between 1 dose is administered and until toxicity occurs that limits its use. administered, at least weekly but can also be fortnightly, monthly, quarterly or annually, intramuscularly, subcutaneously, or intratumorally.

The present invention is further elaborated with the following examples and drawings. However, these examples should not be construed as limiting the scope of the invention.

## BRIEF DESCRIPTION OF THE FIGURES

**Figure 1.** Characterization of the rG-CSF-P64k system by SDS PAGE electrophoresis.

**Figure 2.** Characterization of the rG-CSF-Fc and rGM-CSF-Fc system by SDS PAGE electrophoresis.

**Figure 3.** Kinetic study of the production of anti-G-CSF Ab titers after administration of the therapeutic composition rG-CSF-P64k, alone or in combination with G-CSF.

Figure 4. A) Production of anti-G-CSF antibody titers after administration of the therapeutic composition rG-CSF-P64k, at different doses B) Kinetics of the production of anti-G-CSF antibody titers after administration of the therapeutic composition rG-CSF-P64k, at different doses

**Figure 5.** Evaluation of the circulating neutrophil count after administration of the therapeutic composition rG-CSF-P64k, alone or in combination with G-CSF.

**Figure 6.** Inhibition of cell proliferation induced by sera obtained from mice immunized with the therapeutic composition rGCSF-P64k.

**Figure 7.** Evaluation of the anti-inflammatory effect after the administration of the therapeutic composition rGCSF-P64k in a model of inflammation caused by the application of croton oil. A) Decreased circulating neutrophil count, B) Decreased atrial edema.

**Figure 8.** Kinetic study of the production of Abs anti-G-CSF titers after the administration of the therapeutic composition rG-CSF-Fc, alone or combined with G-CSF.

**Figure 9** A) Studies of the production of anti-G-CSF antibody titers after administration of the therapeutic composition rG-CSF-Fc, at different doses. B) Kinetics of the production of anti-G-CSF antibody titers after administration of the therapeutic composition rG-CSF-Fc, at different doses.

**Figure 10.** Kinetic study of the production of anti-G-CSF antibody titers after the administration of the therapeutic composition rG-CSF-Fc in the Balb/c line

**Figure 11.** Evaluation of circulating neutrophil count after administration of the therapeutic composition rG-CSF-Fc, alone or in combination with G-CSF.

Figure 12. Inhibition of cell proliferation induced by sera obtained from mice immunized with the therapeutic composition rGCSF-Fc.

**Figura 13.** Estudio cinético de producción de titulos de Acs anti-GM-CSF tras la administración de la composición terapéutica rGM-CSF-Fc.

**Figure 14A).** Studies of the production of anti-GM-CSF antibody titers after administration of the therapeutic composition rGM-CSF-Fc, at different doses B) Kinetic studies of the production of anti-GM-CSF antibody titers after administration of the therapeutic composition rGM-CSF-Fc, at different doses.

**Figure 15.** Inhibition of cell proliferation induced by sera obtained from mice immunized with the therapeutic composition rGMCSF-Fc

## EXAMPLES

### Example 1. Obtaining a chemical conjugate between the recombinant protein rP64k and rG-CSF

Obtaining the chemical conjugate between the rGCSF and P64k proteins in a 20:1 ratio begins with 17.6 mg of the GCSF protein, 3 mg of the P64k carrier protein are added in a NaHCO3-Na2CO3 buffer solution (0.01 M) and the solution of 0.5% glutaraldehyde conjugation in a stirred reactor for 1 hour. Subsequently, the details impurities and unconjugated proteins are removed through an ultrafiltration system. This process is carried out using a buffer containing polysorbate 80, sorbitol, sodium acetate, acetic acid, and water for injection. During this operation, 7 buffer changes are performed. The remaining ultrafiltered solution is concentrated to adjust its concentration to the dosage of 1 mg/ml. Later it is stored at 2-8°C.

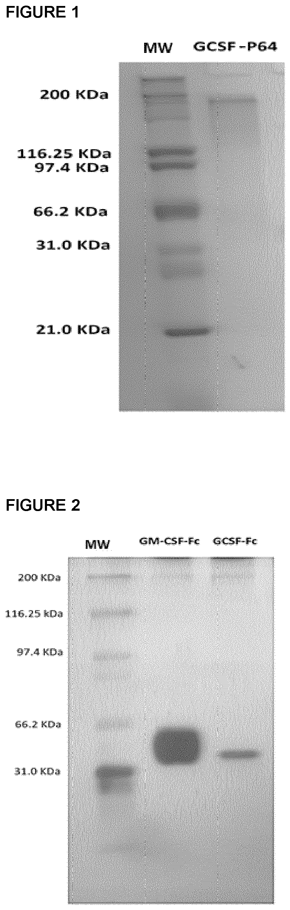

The purity of purified proteins was assessed on 10% SDS-PAGE gel and 1 $\mu$g protein mass. Figure 1 shows that the vaccine composition obtained corresponds approximately to a molecular weight of 200kD.

### Example 2. Obtaining therapeutic compositions through the fusion protein expression method.

A genetic construct based on the G-CSF gene and another with the GM-CSF gene fused to the Fc region of human immunoglobulin G1 and cloned into the PCMX expression vector was designed. Expi 293 cells were transfected with the evaluated genetic construct mixed with polyethyleneimine. Supernatants from transfected cells were collected after 6 days of culture. Said recombinant proteins were purified by affinity to a protein A matrix. The purity of the purified proteins was evaluated in a 7.5% SDS-PAGE gel, the mass of G-CSF-Fc used was 2 $\mu$g and in the case of GM-CSF-Fc was 9 $\mu$g. Figure 2 shows that the molecular weight of both evaluated compositions corresponds approximately to 35 kD.

**Example 3. The therapeutic composition rG-CSF-P64k induces Abs anti-G-CSF.**

Mice of the C57BL/6 line (n=5) were immunized on days 0, 7, 21, 35, and 42 intramuscularly with 50 $\mu$g/Kg of weight of the formulation of Example 1 adjuvanted in Montanide (V/V 1: one). Starting on day 14, they were also administered G-CSF or PBS subcutaneously three times a week.

Blood was drawn to process the serum on days 0 (preimmune), 14, 35, and 56. The titer of specific Abs against G-CSF was determined by ELISA. For this, the plates were covered with 5 $\mu$g/mL of G-CSF and incubated at 37°C for 1 hour. After the corresponding blocking, serum dilutions (1/10, 1/100, 1/500, 1/1000, 1/5000, 1/10000, 1/20000) were added. The reaction was visualized using an anti-murine IgG Abs/alkaline phosphatase conjugate (Sigma), and the corresponding enzyme substrate. The absorbance was read at 405 nm. Pre-immune serum was used as a negative control. The Abs titer was defined as the maximum serum dilution, which showed an ELISA optical density (OD) reading, plus five standard deviations over the mean OD obtained in the wells containing preimmune serum.

The geometric mean of the anti-G-CSF Abs titers evaluated by ELISA of each experimental group was used to define the condition of the immune response.

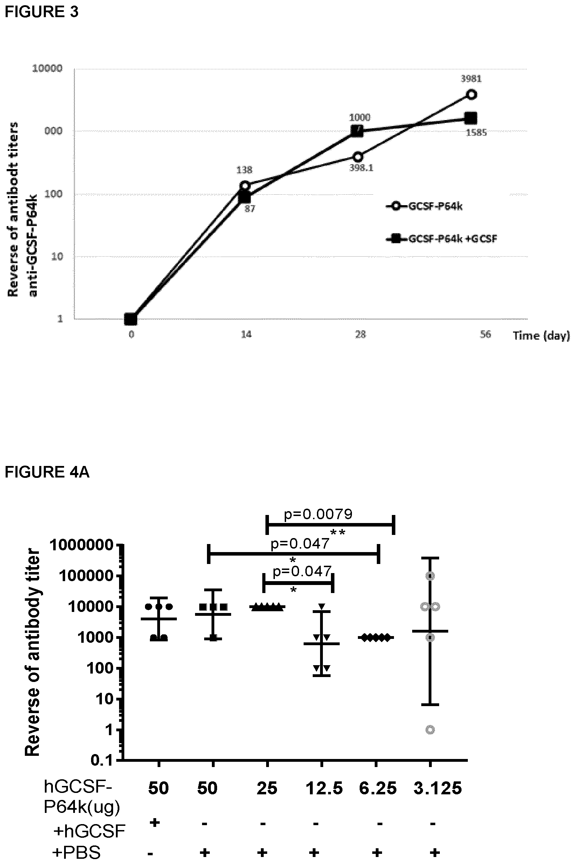

The immunized mice developed specific Abs, which reached titers above 1/10,000, which shows that the vaccine composition induces an immune response against the G-CSF itself (Figure 3).

**Example 4. The therapeutic composition rG-CSF-P64k at different concentrations induces anti-G-CSF antibodies.**

Mice of the C57BL/6 line (n=5) were immunized with 6 doses at intervals of 14 days intramuscularly at different concentrations 50, 25, 12.5, 6.25 and 3.125 $\mu$g of the formulation of Example 1 adjuvanted in Montanide (V/ Ver 1:1). From the second immunization, they were also administered G-CSF or PBS subcutaneously twice a week. Blood was drawn to process the serum on days 0 (pre-immune) and fifteen days after each immunization. The titer of specific antibodies against G-CSF was determined by ELISA. For this, the plates were covered with 5 ug/mL of G-CSF, and incubated at 37°C for 1 hour. After corresponding blocking, serum dilutions (1/100, 1/1000, 1/10000, 1/100000, 1/1000000) were added. The procedure described in Example 3 was then followed.

Immunized mice developed specific antibodies, which reached titers above 1/1000 at different concentrations of the vaccine composition, demonstrating the induction of an immune response against G-CSF. There are significant differences between the titers obtained in the mice immunized with doses of the antigen (FIG. 4A). This demonstrates a dose dependency between the immunized groups with respect to the response obtained measured as antibody titer.

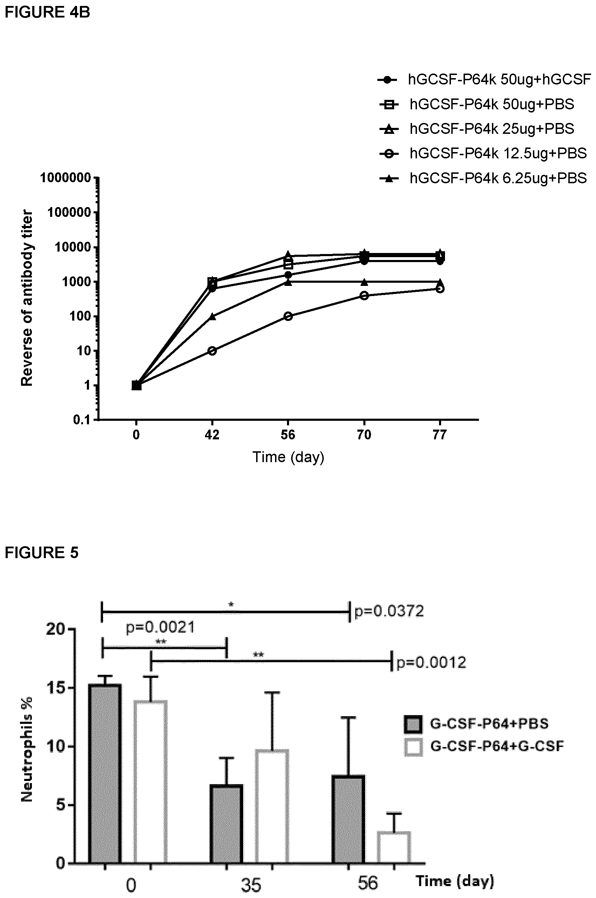

The humoral immune response was also evaluated at different time intervals corresponding to 3, 4, 5 and 6 doses of the vaccine composition. The antibody titer of all the animals immunized at different concentrations of the vaccine preparation increases in a dose-dependent manner, reaching a plateau of the antibody titer from the 5th immunization. (Figure 4B).

**Example 5. The therapeutic composition rG-CSF-P64k decreases the count of circulating neutrophils in C57BL/6 mice.**

C57BL/6 mice (n=5) were immunized with the vaccine formulation of Example 1 adjuvanted in Montanide (V/V 1:1), following the immunization schedule of Example 3. Starting on day 14, they were also administered G- CSF or PBS subcutaneously 3 times a week. On days 0 (pre-immune), and 56 days after the first immunization, peripheral blood was drawn from the maxillary sinus of the animals and collected in vials with EDTA (40 $\mu$l/mL of blood). a Carl Zeiss microscope.

Normality was verified by the Kolmogorov-Smirnov test, the homogeneity of variances by the Levene test and a paired Student's t-test was performed between the values of each animal before immunization and on days 35 and 56. for a significance level of $p < 0.05$. In animals treated with the G-CSF-P64k conjugate, a statistically significant reduction in neutrophils was observed on day 56, suggesting that this pharmaceutical composition is capable of causing neutropenia through the Abs anti-G-CSF that it generates (Figure 5). It should also be noted that neutropenia did not imply a decrease in the survival of the mice.

**Example 6. The therapeutic composition rGCSF-P64k inhibits cell proliferation of the murine myeloblastic line NFS60.**

Using the therapeutic composition of Example 1 and the immunization scheme described in Example 3, the effects of serum obtained at 14 and 28 days were evaluated through a cell proliferation assay in the murine myeloblastic line NFS60 (G-CSF dependent). after immunization of C57BL/6 mice.

The cells were previously thawed and kept in culture for 48 hours to achieve exponential growth. The incubation conditions during the test were temperature 37 °C and atmosphere of 5% CO2. The cells were seeded in 96-well culture plates at a concentration of 10,000 cells per well in the presence of the vaccine formulation whose active ingredient is the rP64k-rG-CSF protein conjugate at 1/250 dilutions; 1/500 and 1/1000. The hG-CSF reference material (MRT (QFB) G-CSF/1905) was used at the same concentrations as a positive control for these assays, and cells with medium without G-CSF were included as a negative control. After 48 hours of incubation, 20 $\mu$L per well of Alamar Blue were added and incubated for 6 hours. Plates were read at 540 and 620 nm. All samples were tested in duplicate.

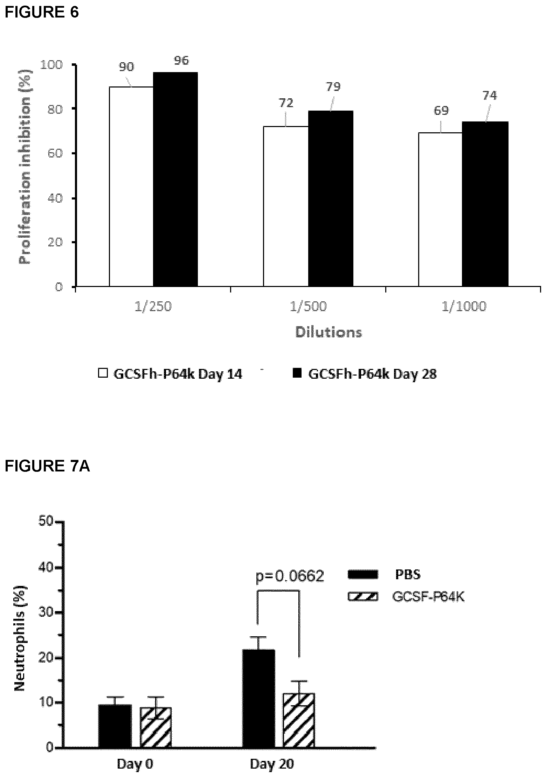

Figure 6 shows that with the administration of the vaccine composition there is an inhibition of proliferation that depends on the dilution (the lower the dilution, the more effect) and the time from the start of a treatment since at 28 days a greater inhibitory effect was observed than at 14 days, which shows that the vaccine formulation inhibits the proliferative effect of G-CSF, which is key in granulopoiesis in the tumor line evaluated.

**Example 7. The therapeutic composition rG-CSF-P64k has an anti-inflammatory effect.**

C57BL/6 mice were immunized subcutaneously on days 0, 7, and 21 with 50 $\mu$g/Kg of weight of the vaccine formulation of Example 1, in Complete Freund's Adjuvant (V:V 1:1). The rest of the immunizations were performed in Freund's Incomplete Adjuvant. As a control group, mice were administered saline solution by the same route and frequency. The animals were applied 10 $\mu$L of 0.4% croton oil topically, on each face of the right ear on days 0, 7, and 21. An equal volume of saline solution was applied to the left ear. On day 0 and day 20, before the start of the croton oil administration, blood samples were taken from all animals for the neutrophil count. On day 35, 4 h after the application of the irritating substance, the animals were sacrificed and the response to ear edema was determined. For them, tissue fragments of 6 mm diameter were taken from the ears and weighed on an analytical balance. With the weights obtained from the discs of both ears, of each animal, the edema produced and the percentage of inhibition of inflammation were calculated, considering:

$$\text{Edema} = \text{Weight of the swollen ear (right ear)} - \text{Weight of the non-swollen ear (left ear)}$$

$$\% \text{ inhibition of inflammation} = [(Pc - Pt)/ Pc] \times 100,$$

where:

Pc: arithmetic means of the change in weight in the control group,
Pt: arithmetic means of the weight variation in the treated group.

The statistical package MINITAB (Minitab Inc. Version 16.1.0. MINITAB, 2010) was used for data processing, establishing a confidence level of 90% in the interpretation of the results. Data were compared between groups, analyzing normality and homogeneity of variance using the Kolmogorov-Smirnov (KS) and Levene's tests, respectively. To determine whether there were significant differences between the experimental groups, the parametric Student's t-test (neutrophil count) and the Mann-Whitney U test for edema were performed.

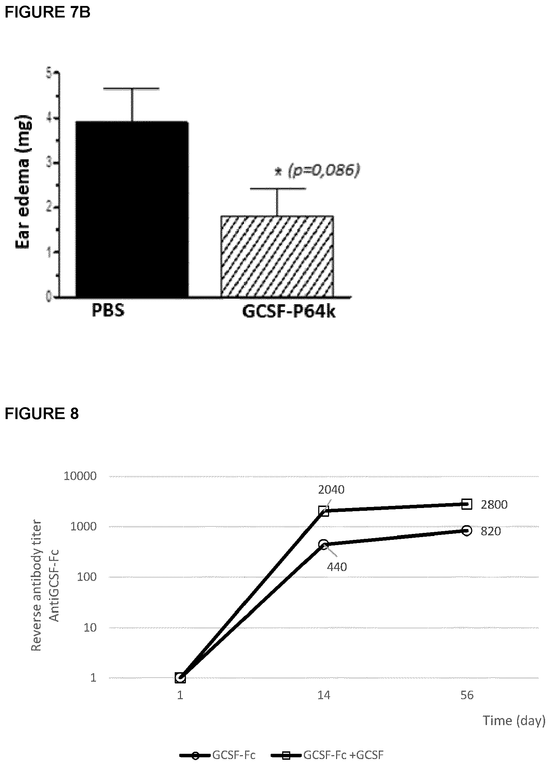

As a result of the previous procedure, on day 20 a statistically significant reduction ($p<0.1$) was detected in the neutrophil count of the group treated with GCSF-P64K conjugate compared to the control group, (Figure 7A) while on day 35 inhibition of acute inflammation in the group treated with GCSF-P64K conjugate compared to the control group, characterized by a statistically significant reduction of 53.85% ($p<0.1$) in-ear edema caused by croton oil (Figure 7B), which indicates that the vaccine preparation has an anti-inflammatory effect in a model of acute inflammation.

**Example 8. The therapeutic composition rG-CSF-Fc induces antibodies response.**

A group of five C57BL/6 mice was immunized intramuscularly on days 0, 14, 28, and 42 with 20 $\mu$g/kg of the vaccine composition described in Example 2, which has G-CSF coupled to Fc as antigen and adjuvanted in Montanide. Blood was drawn to process the serum on days 14 and 56 and specific Abs titers against G-CSF were determined by ELISA. For this, plates were coated with 5 $\mu$g/mL of G-CSFh with a tail of 6 molecules of histidine, from 16 to 20 h at 4 °C. Plates were blocked with 4% skim milk powder diluted in Phosphate Buffered Saline for 1 hour at 25 °C. Serial dilutions of serum from immunized mice and pre-immune serum in blocking solution (1/10-1/107) were added and incubated for 1 hour at 25 °C. 6 washes of 0.1% (V/V) Tween20 solution were performed. An anti-mouse IgG immunoglobulin-horseradish peroxidase conjugate (Sigma, A2554-1 mL) diluted 1:35000 in blocking solution was added and incubated for 1 hour at 25 °C. Orthophenylene was used for color development iamine (0.5 mg/mL) (Sigma, USA) in Substrate Buffer solution ($Na_2HPO_4$ 200 mmol/L, citric acid 100 mmol/L, pH=5) with 0.015% hydrogen peroxide for 30 minutes at 25 °C. The reaction was stopped with 10% $H_2SO_4$. The absorbance was determined at 490 nm in the Dialab GmbH ELISA reader, ELx808.

The titer was determined as the highest dilution for which an absorbance of at least twice the absorbance value of pre-immune serum from the same animal was observed.

The results shown in Figure 8 correspond to the titration of the serum obtained on days 14 and 56. The graph shows that immunization against G-CSFh was able to generate a humoral type response and high titer in mice against said cytokine.

**Example 9. The therapeutic composition rG-CSF-Fc at different concentrations induces anti-G-CSF antibodies.**

Mice of the C57BL/6 line (n=7) were immunized with 6 doses at intervals of 14 days intramuscularly at different concentrations 80, 40, 20, 10 and 5 μg of the formulation of Example 2 that has GCSF as antigen. coupled to Fc and adjuvanted in Montanide (V/V 1:1). Blood was drawn for serum processing on days 0 (pre-immune) and fourteen days after each immunization. The titer of specific antibodies against G-CSF was determined by ELISA and then the procedure of Example 3 was followed.

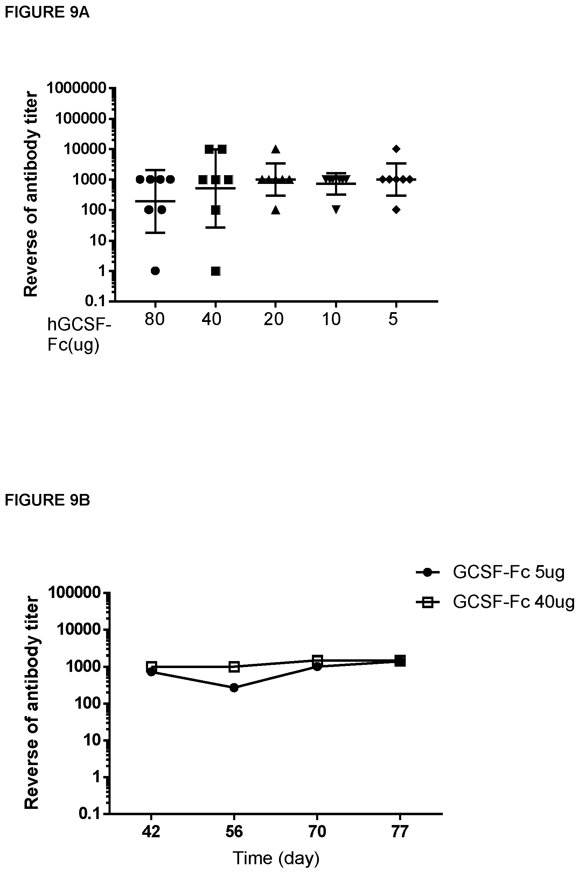

The immunized mice developed specific antibodies, which reached titers above 1/1000 without statistical differences between the groups immunized at different concentrations of the vaccine composition, which demonstrates the induction of an immune response against G-CSF that is not dependent on the dose in the evaluated interval (Figure 9A). The humoral immune response was evaluated at different time intervals, following the procedure of Example 4 for animals immunized with 40 and 5 ug of the vaccine composition. The kinetics of the antibody titer remained in a plateau phase from the 3rd immunization with small variations after the 4th dose. In the development of the humoral response over time, the non-dependence on concentration is maintained (Figure 9B).

Example 10. **The therapeutic composition rG-CSF-Fc in the Balb/c line induces anti-G-CSF antibodies.**

Balb/c mice were immunized with the therapeutic composition of Example 2, which has G-CSF fused to Fc as antigen with 5 doses of 5ug subcutaneously adjuvanted in Montanide, separated at intervals of 14 days. Blood was drawn on days 0 (pre-immune), 42, 70 and 77.

The specific antibody titer against G-CSF was determined by ELISA for the different serum extractions. For this, the plates were covered with 5ug/mL of G-CSF, and incubated at 37°C for 1 hour. After the corresponding blocking, serum dilutions were added (1/100, 1/1000, 1/10,000, 1/100,000, 1/1,000,000) and then proceeded as described in Example 3.

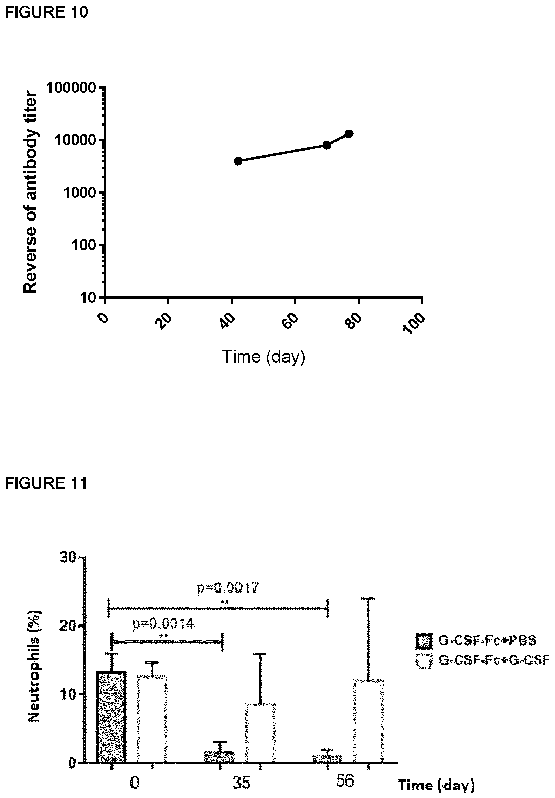

The immunized Balb/c mice developed specific antibodies, which reached mean titers of 1/10,000, demonstrating that the vaccine composition induces a strong immune response against G-CSF (Figure. 10).

**Example 11. The therapeutic composition rG-CSF-Fc decreases the count of circulating neutrophils in C57BL/6 mice.**

C57BL/6 mice (n=5) were administered 20μg/Kg of weight of the vaccine composition described in Example 2, which has G-CSF coupled to Fc as antigen and adjuvanted in Montanide and following the immunization schedule. described in Example 3. Neutrophil count was assessed using a Carl Zeiss microscope. On days 0 (Pre-immune) and 56 of the first immunization, peripheral blood was extracted from the maxillary sinus of the animals and collected in vials with EDTA (40 μl/mL of blood). Neutrophil count was performed using a Carl Zeiss microscope.

Normality was verified by the Kolmogorov-Smirnov test and the homogeneity of variances by the Levene's test, and a paired Student's t-test was performed between the values of each animal before immunization and on days 35 and 56. for a significance level of $p<0.05$.

A statistically significant reduction in neutrophils was observed on day 56 in animals treated with the GCSF-P64k conjugate, suggesting that this pharmaceutical composition is capable of causing neutropenia through the anti-G-CSF Abs it generates (Figure 11). It should also be noted that neutropenia did not imply a decrease in the survival of the mice.

**Example 12. The therapeutic composition rGCSF-Fc inhibits cell proliferation of the murine myeloblastic line NFS60.**

C57BL/6 mice were immunized with the therapeutic composition of Example 2, which has GCSF fused to the Fc as antigen at 40 and 5 ug in 7 intramuscular doses adjuvanted in Montanide, separated at intervals of 14 days.

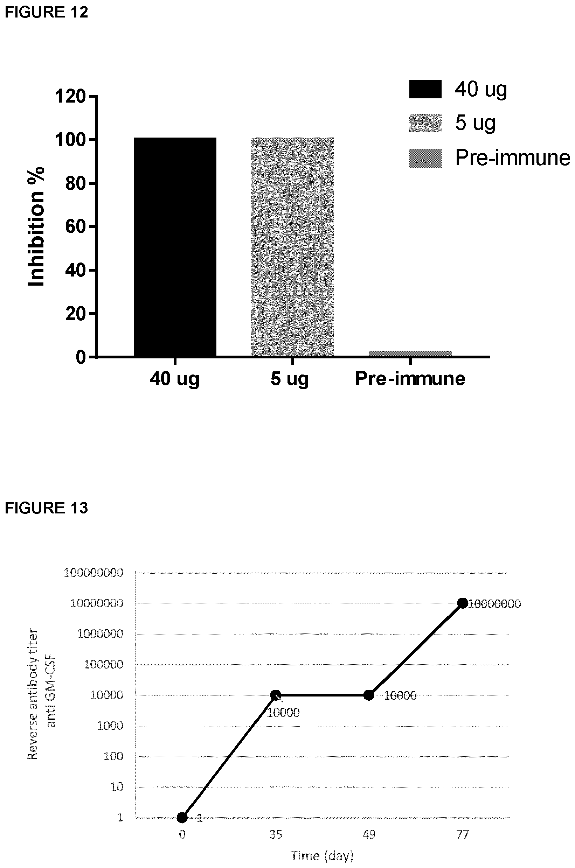

Using a cell proliferation assay in the murine myeloblastic line NFS60 (G-CSF dependent), the effects of the sera obtained on day 0 (pre-immune) and at the end of the immunization scheme were evaluated, and the procedure described in Example 5. Figure 12 shows that with the administration of the vaccine composition proliferation inhibition occurs and not so in the sera of non-immunized animals (pre-immune), which shows that the vaccine formulation inhibits the proliferative effect of the key G-CSF in granulopoiesis in the NFS60 line. The inhibition of the proliferation of the evaluated sera does not show dependence on the concentration of the vaccine composition.

**Example 13. The therapeutic composition rGM-CSF-Fc induces Antibody response.**

A group of four BALB/c mice was immunized with 20 $\mu$g of human GM-CSF fused to an IgG1 Fc chain subcutaneously. Serum was drawn from mice two days before the first immunization to use as a pre-immune serum control. Six immunizations were performed at 14-day intervals, and blood was drawn seven days after the sixth dose. On day 0, immunization was carried out with 20 $\mu$g of the protein emulsified in Freund's Complete Adjuvant (V:V 1:1). The rest of the immunizations were performed in Freund's Incomplete Adjuvant.

On days 0, 35, 49, and 77, blood was drawn and the Abs titer against GM-CSF was determined with the serum by ELISA. For this, 96-well plates were coated with 5 $\mu$g/mL of human GM-CSF dissolved in saline phosphate buffer for 16 to 20 h at 4 °C. Plates were blocked with 4% skim milk powder diluted in saline phosphate buffer for 1 hour at 25 °C. Serial dilutions of serum from immunized mice and pre-immune serum in blocking solution (1/10-1/107) were added and incubated for 1 hour at 25°C. 6 washes of a 0.1% (V:V) Tween 20 solution were performed. An anti-mouse IgG-horseradish peroxidase conjugate diluted 1/35,000 in blocking solution was added and incubated for 1 hour at 25 °C. For color development, orthophenylenediamine (0.5 mg/mL) was used in Substrate Buffer solution (200 mmol/L Na2HPO4, 100 mmol/L citric acid, pH=5) with 0.015% hydrogen peroxide for 30 minutes at 25 °C The reaction was stopped with 10% H2SO4. The absorbance was determined at 490 nm. All samples were applied in duplicate and the titer of antibodies present in the serum of the immunized mice was considered as the highest dilution for which an absorbance of at least twice the absorbance value of the pre-immune serum of the same animal was observed.

In Figure 13, the graph shows that immunization against human GM-CSF generated a high response with titers of up to 1x107. The results indicate that the immunization was capable of generating a high titer humoral response in the mice against such cytokine.

**Example 14. The therapeutic composition rGM-CSF-Fc at different concentrations induces anti-GM-CSF antibodies.**

Mice of the C57BL/6 line (n=7) were immunized with 6 doses at intervals of 14 days intramuscularly at different concentrations 40, 20, 10, 5 and 2.5 $\mu$g of the formulation of Example 2 that has GMCSF as antigen coupled to Fc and adjuvanted in Montanide (V/V 1:1).

Blood was drawn for serum processing on days 0 (pre-immune) and fourteen days after each immunization. The titer of specific antibodies against GM-CSF was determined by ELISA. For this, the plates were covered with 5 $\mu$g/mL of GM-CSF, incubated at 37°C for 1 hour, following the procedure of Example 4.

The geometric mean of the anti-GM-CSF antibody titers evaluated by ELISA of each experimental group was used to define the condition of the immune response.

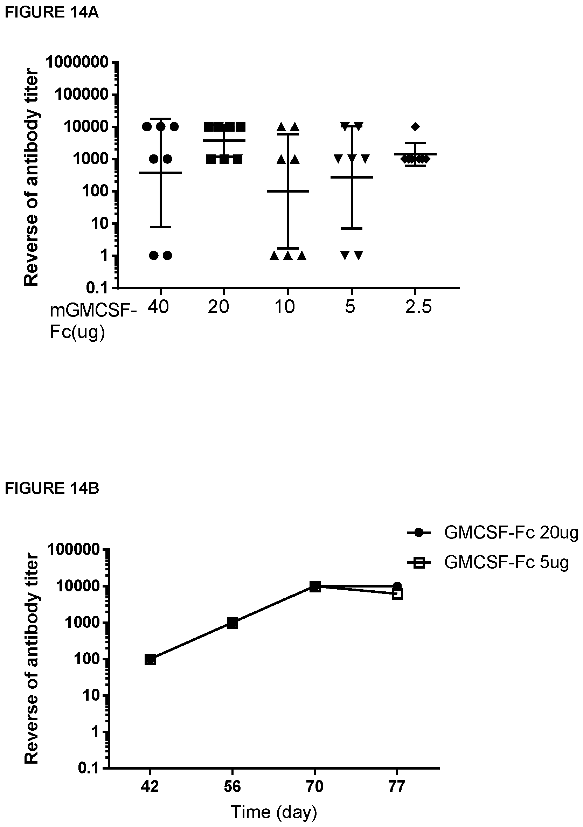

The immunized mice developed specific antibodies, which reached titers above 1/1000 without statistical differences between the groups immunized at different concentrations of the vaccine composition, which demonstrates the induction of an immune response against GM-CSF (Figure 14A) that in the dose range tested is not concentration dependent.

The humoral immune response was evaluated at different time intervals, following the procedure of Example 4 for animals immunized with 20 and 5 ug of the vaccine composition. The kinetics of the antibody titer increases in correspondence with the number of doses. From the 5th dose, a plateau phase is observed where there are no variations in the response. In the development of the humoral response over time, the non-dependence on concentration is maintained (FIG. 14B).

**Example 15 The therapeutic composition rGMCSF-Fc inhibits cell proliferation of the murine myeloblastic line NFS60.**

Balb/c mice were immunized with the therapeutic composition of Example 2, which has GMCSF fused to the Fc as antigen with 5 doses of 5ug subcutaneously adjuvanted in Montanide, separated at intervals of 14 days. Blood was drawn on day 0 (pre-immune) and 14 days after the last immunization.

The effects of the sera obtained from the immunization were evaluated by means of a cell proliferation assay in the murine myeloblastic line NFS60 (G-CSF dependent), and the same procedure described in Example 6 was followed.

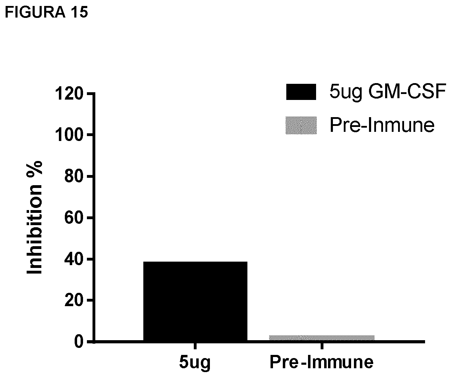

Figure 15 shows that with the administration of the vaccine composition rGMCSF-Fc there is inhibition of proliferation in immunized animals and not in the sera of non-immunized animals (pre-immune), which shows that the vaccine formulation inhibits the effect proliferation of G-CSF key in granulopoiesis in the NFS60 line

## Claims

1. A therapeutic vaccine composition to induce an immune response against hematopoietic growth factors comprising a carrier protein, an adjuvant and at least one antigen selected from the group comprising of:

   - recombinant granulocyte colony-stimulating factor (rG-CSF) and
   - recombinant granulocyte-macrophage colony-stimulating factor (rGM-CSF).

2. The vaccine composition of claim 1 wherein the carrier protein is selected from the group comprising:

   - cholera toxin B subunit,
   - tetanus toxoid,
   - KLH,
   - P64k of Neisseria meningitidis,
   - diphtheria toxoid,
   - peptides able to presenting epitopes against G-CSF and GM-CSF,
   - immunoglobulin G,
   - immunoglobulin M,
   - antibody Fc region,
   - antibody variable region,
   - bacteria proteins,
   - yeast proteins and
   - mammalian proteins.

3. The vaccine composition according to any of claims 1-2 wherein the carrier protein is bounded to the antigen by any of the following methods:

   - chemical conjugation and
   - fusion.

4. The vaccine composition according to any of claims 1-3 wherein the antigen is rG-CSF.

5. The vaccine composition according to any of claims 1-3 wherein the antigen is rGM-CSF.

6. The vaccine composition according to any of claims 1-3 wherein the adjuvant is selected from the group comprising of:

   - incomplete Freund's adjuvants,
   - complete Freund's adjuvants,
   - squalene-based adjuvants,
   - synthetic origin adjuvants,
   - mineral origin adjuvants,
   - vegetable origin adjuvants,
   - animal origin adjuvants,
   - particulated proteic adjuvants,
   - proteoliposomes-type adjuvants,

- liposomes and
- a mix of the above adjuvants.

7. Use of the vaccine composition of any of claims 1-6 in the treatment of inflammatory diseases selected from the group comprising of:

- cancer,
- chronic obstructive pulmonary disease,
- uveitis
- rheumatoid arthritis,
- ankylosing spondylitis,
- lupus erythematosus,
- Crohn disease,
- asthma,
- dermatitis,
- cytokine release syndrome
- diseases where cellular degranulation is relevant.

8. A method of treatment in a subject in need comprising the administration of a therapeutic effective amount in a range between 0.01 y 10 mg/kg of weigh of therapeutic vaccine composition of any of claims 1-6.

9. The method according to claim 8 wherein an immune response induction stage is achieved with one to six doses of the vaccine composition, at least weekly administered and at least one dose and until use limiting toxicity occurs as maintenance weekly administered.

10. The method according to claim 9 wherein the administration route of the vaccine composition is selected from the group comprising: intramuscular, subcutaneous and intratumoral.

## FIGURE 1

## FIGURE 2

FIGURE 3

FIGURE 4A

FIGURE 4B

FIGURE 5

FIGURE 6

FIGURE 7A

FIGURE 7B

FIGURE 8

FIGURE 9A

FIGURE 9B

FIGURE 10

FIGURE 11

FIGURE 12

FIGURE 13

FIGURE 14A

FIGURE 14B

FIGURA 15

## INTERNATIONAL SEARCH REPORT

| International application No |
|---|
| PCT/CU2022/050002 |

**A. CLASSIFICATION OF SUBJECT MATTER**
INV. A61K39/00 A61P37/00 C07K14/535
ADD.

According to International Patent Classification (IPC) or to both national classification and IPC

**B. FIELDS SEARCHED**

Minimum documentation searched (classification system followed by classification symbols)
A61K A61P C07K

Documentation searched other than minimum documentation to the extent that such documents are included in the fields searched

Electronic data base consulted during the international search (name of data base and, where practicable, search terms used)

EPO-Internal

**C. DOCUMENTS CONSIDERED TO BE RELEVANT**

| Category* | Citation of document, with indication, where appropriate, of the relevant passages | Relevant to claim No. |
|---|---|---|
| Y | CORNISH A L ET AL: "G-CSF and GM-CSF as therapeutic targets in rheumatoid arthritis", NATURE REVIEWS RHEUMATOLOGY, NATURE PUBLISHING GROUP, GB, vol. 5, no. 10, 1 January 2009 (2009-01-01), pages 554-559, XP009169791, ISSN: 1759-4790, DOI: 10.1038/NRRHEUM.2009.178 abstract page 3, paragraph 4 – page 4, paragraph 1 ----- | 1-10 |
| Y | WO 2018/145206 A1 (ME THERAPEUTICS INC [CA]) 16 August 2018 (2018-08-16) abstract claims 1-3,21,25-26,28 page 1, paragraph 3 – page 2, paragraph 4 ----- | 1-10 |

–/––

| X | Further documents are listed in the continuation of Box C. | | X | See patent family annex. |
|---|---|---|---|---|

* Special categories of cited documents :

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance;; the claimed invention cannot be considered novel or cannot be considered to involve an inventive step when the document is taken alone

"Y" document of particular relevance;; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 19 August 2022 | 05/09/2022 |

| Name and mailing address of the ISA/ European Patent Office, P.B. 5818 Patentlaan 2 NL - 2280 HV Rijswijk Tel. (+31-70) 340-2040, Fax: (+31-70) 340-3016 | Authorized officer Kapeller, Anita |
|---|---|

Form PCT/ISA/210 (second sheet) (April 2005)

## INTERNATIONAL SEARCH REPORT

| | International application No |
|---|---|
| | PCT/CU2022/050002 |

| C(Continuation). | DOCUMENTS CONSIDERED TO BE RELEVANT | |
|---|---|---|
| Category* | Citation of document, with indication, where appropriate, of the relevant passages | Relevant to claim No. |
| Y | EP 1 829 893 A1 (CENTRO INMUNOLOGIA MOLECULAR [CU]) 5 September 2007 (2007-09-05) abstract claims 1-5,15-17 ----- | 1-10 |

Form PCT/ISA/210 (continuation of second sheet) (April 2005)

## INTERNATIONAL SEARCH REPORT

Information on patent family members

International application No

PCT/CU2022/050002

| Patent document cited in search report | | Publication date | Patent family member(s) | | Publication date |
|---|---|---|---|---|---|
| WO 2018145206 | A1 | 16-08-2018 | CA | 3052877 A1 | 16-08-2018 |
| | | | CN | 110382532 A | 25-10-2019 |
| | | | EP | 3580236 A1 | 18-12-2019 |
| | | | JP | 2020507339 A | 12-03-2020 |
| | | | WO | 2018145206 A1 | 16-08-2018 |
| EP 1829893 | A1 | 05-09-2007 | AR | 051494 A1 | 17-01-2007 |
| | | | AT | 525393 T | 15-10-2011 |
| | | | AU | 2005306186 A1 | 26-05-2006 |
| | | | BR | PI0518007 A | 21-10-2008 |
| | | | CA | 2588573 A1 | 26-05-2006 |
| | | | CN | 101061136 A | 24-10-2007 |
| | | | CR | 9097 A | 28-08-2007 |
| | | | CU | 23297 A1 | 24-07-2008 |
| | | | EA | 200701071 A1 | 28-12-2007 |
| | | | EP | 1829893 A1 | 05-09-2007 |
| | | | EP | 2112160 A2 | 28-10-2009 |
| | | | GE | P20105039 B | 12-07-2010 |
| | | | HK | 1113374 A1 | 03-10-2008 |
| | | | JP | 5227028 B2 | 03-07-2013 |
| | | | JP | 2008526686 A | 24-07-2008 |
| | | | KR | 20070084025 A | 24-08-2007 |
| | | | MA | 29044 B1 | 01-11-2007 |
| | | | MY | 162106 A | 31-05-2017 |
| | | | PE | 20061172 A1 | 16-12-2006 |
| | | | SG | 122897 A1 | 29-06-2006 |
| | | | TN | SN07187 A1 | 21-11-2008 |
| | | | TR | 200704213 T2 | 21-08-2007 |
| | | | TW | I433853 B | 11-04-2014 |
| | | | US | 2009274647 A1 | 05-11-2009 |
| | | | WO | 2006053508 A1 | 26-05-2006 |
| | | | ZA | 200703528 B | 25-06-2008 |

Form PCT/ISA/210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019178645 A
- WO 2020113270 A
- WO 2010124163 A
- WO 2019070680 A

### Non-patent literature cited in the description

- **ROGOVSKII V.** *Front Immunol.,* 2020, vol. 11, 2061
- **STAPE T.** *Asia Pac J Oncol Nurs,* 2018, vol. 5, 40-2
- **WATARI K et al.** *Blood,* 1989, vol. 73 (1), 117-22
- **HAMILTON JA.** *J Exp Med.,* 2020, vol. 217 (1), e20190945
- **EYLES JL et al.** *Nat. Clin. Pract. Rheumatol.,* 2006, vol. 2 (9), 500-510
- **AHANDIDEH B et al.** *Hum Immunol.,* 2020, vol. 81 (5), 206-217
- **DO H et al.** *Cancers,* 2020, vol. 12 (2), 287
- **SILVA EM et al.** *PLoS ONE,* 2017, vol. 12 (7), e0181125
- **LIPPITZ, BE.** *The Lancet Oncology,* 2013, vol. 14 (6), e218-e228
- **LEE HN et al.** *Rheumatol Int.,* 2019, vol. 39 (5), 859-868
- **ROBERTS, AW.** *Growth Factors,* 2005, vol. 23 (1), 33-4
- **MEHTA HM et al.** *J. Immunol.,* 2015, vol. 195 (4), 1341-1349
- **CAMPBELL IK.** *J. Immunol.,* 2016, vol. 197, 4392-4402